# EUROPÄISCHE PATENTSCHRIFT

(11) **EP 1 504 274 B1**
(45) Veröffentlichungstag und Bekanntmachung des Hinweises auf die Patenterteilung: **05.03.2008**
(21) Anmeldenummer: 03735279.6
(22) Anmeldetag: 06.05.2003
(51) Int. Cl.: G01R 33/34

(54) **MAGNETRESONANZANLAGE**
MAGNETIC RESONANCE SYSTEM
APPAREIL A RESONANCE MAGNETIQUE

(30) Priorität: 15.05.2002 DE 10221636
(43) Veröffentlichungstag der Anmeldung: 09.02.2005
(73) Patentinhaber: SIEMENS AKTIENGESELLSCHAFT, 80333 München (DE)
(72) Erfinder: VESTER, Markus, 90471 Nürnberg (DE); RENZ, Wolfgang, 91052 Erlangen (DE); NISTLER, Jürgen, 91056 Erlangen (DE); LAZAR, Razvan, 91056 Erlangen (DE)
(86) Internationale Anmeldenummer: PCT/DE2003/001444
(87) Internationale Veröffentlichungsnummer: WO 2003/098247

(56) Entgegenhaltungen:
- US-A- 4 682 112
- US-A- 5 216 367
- US-A- 5 642 048
- FOO T K F ET AL: "REDUCTION OF RF PENETRATION EFFECTS IN HIGH FIELD IMAGING" MAGNETIC RESONANCE IN MEDICINE, ACADEMIC PRESS, DULUTH, MN, US, Bd. 23, 1992, Seiten 287-301, XP000261762 ISSN: 0740-3194

## Beschreibung

Die vorliegende Erfindung betrifft eine Magnetresonanzanlage mit einem Grundfeldmagneten, einem Gradientensystem, einer Primärsendeantenne, mindestens einer Sekundärsendeantenne, mindestens einer Empfangsantenne und einer Steuer- und Auswerteeinrichtung,
- wobei ein Untersuchungsbereich mittels des Grundfeldmagneten mit einem statischen Grundmagnetfeld beaufschlagbar ist,
- wobei der Untersuchungsbereich mittels des Gradientensystems mit Gradientenmagnetfeldern beaufschlagbar ist,
- wobei der Untersuchungsbereich mittels der Sendeantennen mit hochfrequenten Anregungsfeldern beaufschlagbar ist,
- wobei mittels der Empfangsantenne Magnetresonanzsignale empfangbar sind, zu denen ein in den Untersuchungsbereich einbringbares Untersuchungsobjekt anregbar ist,
- wobei das Gradientensystem und die Sendeantennen von der Steuer- und Auswerteeinrichtung ansteuerbar sind, so dass im Gradientensystem Gradientenströme fließen und in den Sendeantennen Anregungsströme fließen,
- wobei die Magnetresonanzsignale der Steuer- und Auswerteeinrichtung zuführbar und von dieser auswertbar sind,
- wobei der Sekundärsendeantenne mindestens ein Steuerelement zugeordnet ist, mittels dessen ein Amplitudenverhältnis und/oder ein Phasenversatz der Anregungsströme zueinander einstellbar ist.

Magnetresonanzanlagen sind allgemein bekannt. Beispielhaft wird auf das Fachbuch "Bildgebende Systeme für die medizinische Diagnostik", 3. Auflage, 1995, publicis MCD Verlag, Seiten 501 bis 503, verwiesen.

Diese bekannte Resonanzanlage weist ein Grundmagnetfeld auf, das in der Regel 1,5 T beträgt. Mit dieser Magnetresonanzanlage ist eine gute Rekonstruktion des Untersuchungsobjekts möglich.

In jüngerer Zeit werden auch Magnetresonanzanlagen hergestellt, bei denen das Grundmagnetfeld mehr als 1,5 T, insbesondere 3 T und mehr, aufweist. Mit diesen Magnetresonanzanlagen sind bessere Auflösungen erzielbar. Bei derartigen Magnetresonanzanlagen treten im Stand der Technik aber vermehrt Bildinhomogenitäten auf, die von Hochfrequenz-Wirbelströmen im Untersuchungsobjekt herrühren.

Im Stand der Technik - hier z. B. der US 6,252,403 B1 - wird zur Kompensation derartiger Wirbelströme vorgeschlagen, die Sendeantenne spiralförmig auszubilden. Die Sendeantenne nimmt also die Gestalt eines um seine Symmetrieachse verwundenen Birdcage-Resonators an. Aus der genannten US-Patentschrift ist ferner schon bekannt, in der Nähe des Untersuchungsobjekts geeignete Dielektrika, insbesondere Wasser, anzuordnen, um hierdurch eine Homogenisierung des hochfrequenten Anregungsfeldes zu erreichen. Trotz aller Bemühungen des Standes der Technik können die Inhomogenitäten im Anregungsfeld aber nicht in allen Fällen hinreichend kompensiert werden.

Aus der US-A-4,682,112 ist eine Magnetresonanzanlage nach dem Oberbegriff des Anspruchs 1 bekannt. Bei dieser Magnetresonanzanlage besteht die Sendeanordnung aus einem Array gleich ausgebildeter Sendeantennen, die axial und/oder in Umfangsrichtung voneinander beabstandet sind. Sie werden von der Steuer- und Auswerteeinrichtung einzeln angesteuert, um eine täumlich begrenzte HF-Anregung zu erreichen.

Aus der US-A-5,216,367 ist eine Magnetresonanzanlage bekannt, bei der mehrere einzeln ansteuerbare Empfangsantennen vorgesehen sind.

Aus der US-A-5,642,048 ist eine Magnetresonanzanlage bekannt, bei der die Sendeantenne als modifizierter Birdcage-Resonator ausgebildet ist.

Aus dem Fachaufgatz "Reduclion of RF Penetration Effects in High Field Imaging' von Thomas F. Foo, Cecil E. Hayes und Yoon-Won Kang, erschienen in Magnetic Resonance in Medicine 23 (1992), Heft 2, Seiten 287 bis 301, ist bekannt, die dielektrische Kopplung des Hochfrequenzschirms einer Ganzkörpersendeantenne zu verändern, um ein möglichst homogenes Hochfrequenzfeld innerhalb eines Anregungsvolumens zu erreichen.

Die Aufgabe der vorliegenden Erfindung besteht darin, eine einfach ausgebildete Magnetresonanzanlage zu schaffen, bei der das Anregungsfeld trotz eines hohen Grundmagnetfeldes im Untersuchungsobjekt möglichst homogen ist, und dies zu erreichen ohne manuelle Eingriffe an einem Dielektrikum etc wie in obigem Fachaufsatz.

Die Aufgabe wird dadurch gelöst, dass der in der Sekundärsendeantenne fließende Anregungsstrom eine kleinere Amplitude aufweist als der in der Primärsendeantenne fließende Anregungsstrom und dass das von der Sekundärsendeantenne erzeugte Anregungsfeld eine kleinere räumliche Erstreckung aufweist als das von der Primärsendeantenne erzeugte Anregungsfeld.

Die Sekundärsendeantenne wirkt also vorzugsweise nur lokal zur Korrektur des von der Primärsendeantenne erzeugten Anregungsfeldes. Durch diese Ausgestaltung ist das von der Primärsendeantenne erzeugte Anregungsfeld somit gezielt und aktiv homogenisierbar. Es wird also das mindestens eine Steuerelement derart angesteuert, dass die Überlagerung der Anregungsfelder bei in den Untersuchungsbereich eingebrachtem Untersuchungsobjekt im Untersuchungsobjekt möglichst homogen ist.

Es ist möglich, dass die Sekundärsendeantenne völlig unabhängig von der Primärsendeantenne betreibbar ist. Vorzugsweise aber ist sie induktiv mit der Primärsendeantenne gekoppelt.

Wenn der Steuer- und Auswerteeinrichtung ein Steuersignal vorgebbar ist, von dem die Ansteuerung des Steuerelements abhängig ist, ist eine Anpassung an das Untersuchungsobjekt möglich. Das Steuersignal kann beispielsweise implizit durch Körpergröße und Gewicht, evtl. auch weitere Charakteristika, eines zu untersuchenden Patienten bestimmt sein. Alternativ ist die Ansteuerung auch durch Versuche ermittelbar.

Im Einzelfall kann es auch sinnvoll sein, wenn die Sekundärsendeantenne in Abhängigkeit vom Steuersignal auch derart betreibbar ist, dass sie bei einer Magnetresonanzfrequenz nicht resonant ist und den Untersuchungsbereich nicht mit einem Anregungsfeld beaufschlagt. Die Sekundärsendeantenne kann also im Einzelfall auch schlichtweg abschaltbar sein.

Wenn das Steuerelement als einstellbare Impedanz ausgebildet ist, sind Amplitudenverhältnis und/oder Phasenversatz der Anregungsströme besonders flexibel einstellbar.

Der in der Sekundärsendeantenne fließende Anregungsstrom kann zum in der Primärsendeantenne fließenden Anregungsstrom einen Phasenversatz zwischen -180° und +180° elektrisch aufweisen.

Es ist möglich, dass die Sekundärsendeantenne mit der Steuer- und Auswerteeinrichtung derart verbunden ist, dass sie nicht zum Empfang der Magnetresonanzsignale nutzbar ist. In diesem Fall wird die Sekundärsendeantenne also ausschließlich zur Homogenisierung des Anregungsfeldes eingesetzt. Es ist aber auch möglich, dass die Sekundärsendeantenne auch zum Empfang der Magnetresonanzsignale nutzbar ist. In diesem Fall ist sie also vorzugsweise als sogenannte Lokalspule ausgebildet, die üblicherweise zum lokalen Empfang von Magnetresonanzsignalen bereits genutzt werden. Insbesondere in diesem Fall ist die Sekundärsendeantenne beim Empfang von Magnetresonanzsignalen von der Steuer- und Auswerteeinrichtung derart abstimmbar, dass sie bei einer Magnetresonanzfrequenz resonant ist.

Weitere Vorteile und Einzelheiten ergeben sich aus der nachfolgenden Beschreibung eines Ausführungsbeispiels in Verbindung mit den Zeichnungen. Dabei zeigen in Prinzipdarstellung
- FIG 1: eine Magnetresonanzanlage,
- FIG 2: eine Primärsendeantenne und eine Sekundärsendeantenne,
- FIG 3: eine Sekundärsendeantenne und
- FIG 4: eine Primärsendeantenne und eine Sekundärsendeantenne.

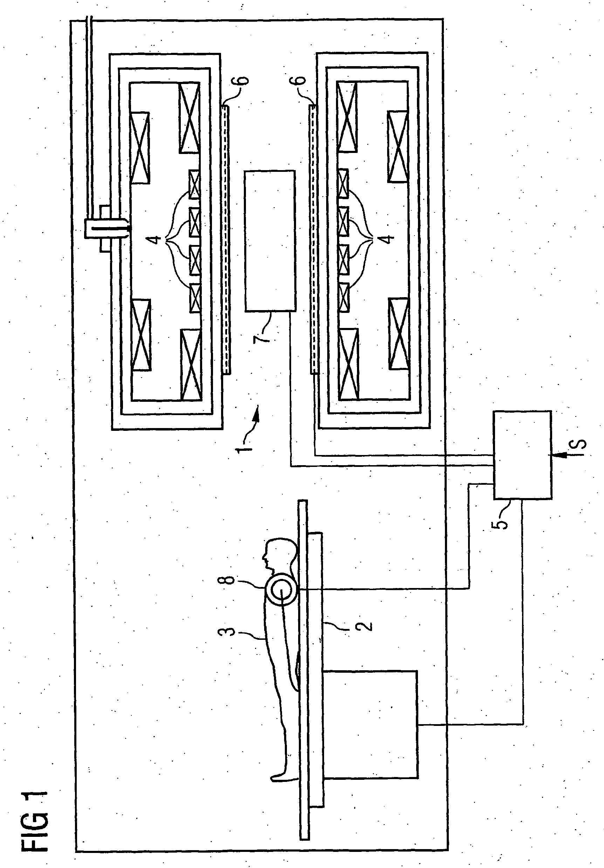

Gemäß FIG 1 weist ein Magnetresonanzanlage einen Untersuchungsbereich 1 auf. Mittels einer Patientenliege 2 ist ein Untersuchungsobjekt 3, hier ein Mensch 3, in den Untersuchungsbereich 1 einbringbar.

Der Untersuchungsbereich 1 wird mittels eines Grundfeldmagneten 4 mit einem Grundmagnetfeld beaufschlagt. Das Grundmagnetfeld ist zeitlich konstant (statisch) und örtlich so homogen wie möglich. Es weist eine magnetische Feldstärke auf, die größer als 1,5 T ist. Vorzugsweise liegt sie bei 3 T oder mehr.

Der Grundfeldmagnet 4 ist vorzugsweise supraleitend ausgebildet. Es ist somit keine weitere Ansteuerung und/oder Anlenkung durch eine Steuer- und Auswerteeinrichtung 5 erforderlich.

Die Magnetresonanzanlage weist ferner ein Gradientensystem 6 auf, mittels dessen der Untersuchungsbereich 1 mit Gradientenmagnetfeldern beaufschlagbar ist. Das Gradientensystem 6 ist von der Steuer- und Auswerteeinrichtung 5 ansteuerbar, so dass im Gradientensystem 6 Gradientenströme fließen.

Die Magnetresonanzanlage weist auch eine Primärantenne 7 und eine Sekundärantenne 8 auf. Ersichtlich ist die Primärantenne 7 als Ganzkörperantenne 7 ausgebildet. Die Sekundärantenne 8 hingegen ist als Lokalspule 8 ausgebildet. Sie wirkt also nur lokal. Die Antennen 7, 8 sind von der Steuer- und Auswerteeinrichtung 5 ansteuerbar, so dass in den Antennen 7, 8 Anregungsströme fließen.

Mittels der Primärantenne 7 ist der Untersuchungsbereich 1 mit einem hochfrequenten Anregungsfeld beaufschlagbar. Falls in den Untersuchungsbereich 1 das Untersuchungsobjekt 3 eingebracht ist, ist dieses somit zu Magnetresonanzen anregbar. Die so erzeugten Magnetresonanzsignale sind dann wahlweise mittels der Primärantenne 7 oder der Sekundärantenne 8 empfangbar. Beide Antennen 7, 8 stellen also Empfangsantennen 7, 8 der Magnetresonanzanlage dar. Die empfangenen Magnetresonanzsignale sind der Steuer- und Auswerteeinrichtung 5 zuführbar und von dieser auswertbar.

Aufgrund des hohen Grundmagnetfeldes von 3 T und mehr ist es nur mittels der Primärantenne 7 nicht in allen Fällen möglich, im gesamten Untersuchungsobjekt 3 ein homogenes Anregungsfeld zu erzeugen. Daher ist auch die Sekundärantenne 8 von der Steuer- und Auswerteeinrichtung 5 ansteuerbar, so dass sie das Untersuchungsobjekt 3 zumindest lokal ebenfalls mit einem hochfrequenten Anregungsfeld beaufschlagt. Das von der Sekundärantenne 8 erzeugte Anregungsfeld weist aber eine kleinere räumliche Erstreckung auf als das von der Primärantenne 7 erzeugte Anregungsfeld. Ferner weist der in der Sekundärantenne 8 fließende Anregungsstrom auch eine kleinere Amplitude als der in der Primärantenne 7 fließende Anregungsstrom auf.

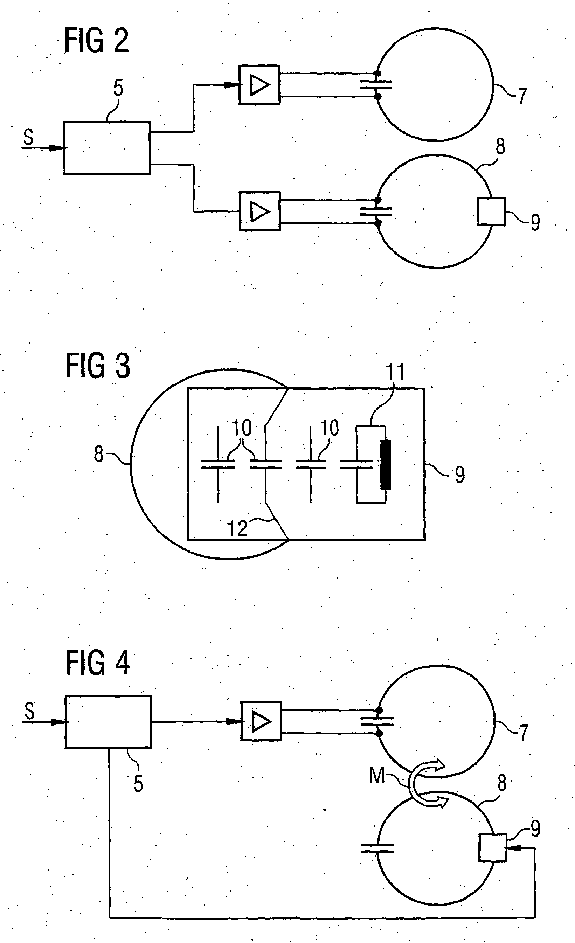

Gemäß FIG 2 ist die Sekundärantenne 8 von der Steuer- und Auswerteeinrichtung 5 unabhängig von der Primärsendeantenne 7 ansteuerbar. Die Steuer- und Auswerteeinrichtung 5 stellt somit selbst ein Steuerelement dar, mittels dessen ein Amplitudenverhältnis und/oder ein Phasenversatz der in den Antennen 7, 8 fließenden Anregungsströme einstellbar ist.

Die Ansteuerung der Sekundärantenne 8 durch die Steuer- und Auswerteeinrichtung 5 kann insbesondere derart erfolgen, dass der Phasenversatz zwischen -180° und +180° elektrisch liegt. Er ist also beliebig einstellbar. Auch das Amplitudenverhältnis ist - innerhalb der Auslegungsgrenzen der Sekundärantenne 8 - beliebig einstellbar. Insbesondere kann die Sekundärantenne 8 im Einzelfall auch derart betrieben werden, dass sie den Untersuchungsbereich 1 nicht mit einem Anregungsfeld beaufschlagt. Der in der Sekundärantenne 8 fließende Anregungsstrom hat in diesem Fall also den Wert 0.

In der Sekundärantenne 8 ist ein Steuerelement 9 angeordnet. Mittels des Steuerelements 9 wird, falls die Sekundärantenne 8 den Untersuchungsbereich 1 nicht mit einem Anregungsfeld beaufschlagen soll, die Sekundärantenne 8 verstimmt, so dass sie bei einer Magnetresonanzfrequenz nicht resonant ist.

Die Ansteuerung der Sekundärantenne 8 erfolgt in Abhängigkeit von einem Steuersignal S, das der Steuer- und Auswerteeinrichtung 5 durch einen Anwender von außen vorgegeben wird. Die Ansteuerung des Steuerelements 9 ist also vom Steuersignal S abhängig.

Gemäß FIG 3 weist das Steuerelement 9 mehrere Kapazitäten 10 und einen Parallelschwingkreis 11 auf. Über einen Schalter 12 wird in Abhängigkeit vom Steuersignal S eine der Kapazitäten 10 oder der Parallelschwingkreis 11 zugeschaltet. Das Steuerelement 9 ist also als einstellbare Impedanz 9 ausgebildet.

Beim Zuschalten einer der Kapazitäten 10 erfolgt - je nach zugeschalteter Kapazität 10 - eine Anhebung oder eine Abschwächung des von der Primärsendeantenne 7 erzeugten Anregungsfeldes oder eine exakte Abstimmung der Sekundärantenne 8 auf die Magnetresonanzfrequenz. Das Abstimmen auf die Magnetresonanzfrequenz wird nur im Empfangsfall vorgenommen.

Wird der Parallelschwingkreis 11 zugeschaltet, ist die Sekundärantenne 8 verstimmt, so dass sie bei der Magnetresonanzfrequenz nicht mehr anregbar ist. Dies entspricht einem Wegschalten der Sekundärantenne 8, wenn diese also im Sende- oder Empfangsfall überhaupt nicht genutzt werden soll.

Die Darstellung gemäß FIG 4 entspricht im wesentlichen der von FIG 2. Bei FIG 4 erfolgt aber keine separate Anregung der Sekundärantenne 8. Vielmehr besteht zwischen den Antennen 7, 8 eine induktive Kopplung M. Die Sekundärantenne 8 ist mit der Primärantenne 7 also induktiv gekoppelt. In diesem Fall erfolgt mittels des Steuerelements 9 nicht nur eine Einstellung der Resonanzfrequenz der Sekundärantenne 8, sondern mit dem Einstellen der Resonanzfrequenz indirekt auch ein Einstellen von Amplitude und Phasenversatz des in der Sekundärantenne 8 fließenden Anregungsstroms relativ zum in der Primärantenne 7 fließenden Anregungsstrom.

Sowohl bei der Ausführungsform gemäß FIG 2 als auch bei der Ausführungsform gemäß FIG 4 erfolgt die Ansteuerung des Steuerelements 9 und - direkt oder indirekt - der Sekundärantenne 8 derart, dass die Überlagerung der Anregungsfelder im Untersuchungsobjekt 3 möglichst homogen ist.

Beim Empfang von Magnetresonanzsignalen mittels der Sekundärantenne 8 wird diese von der Steuer- und Auswerteeinrichtung 5 stets derart abgestimmt, dass sie bei der Magnetresonanzfrequenz resonant ist.

Obenstehend wurde beschrieben, dass die Sekundärantenne 8 sowohl zum Beaufschlagen des Untersuchungsbereichs 1 mit einem Anregungsfeld als auch zum Empfangen von Magnetresonanzsignalen genutzt wird. Es ist selbstverständlich auch möglich, die Sekundärantenne 8 ausschließlich zum Beaufschlagen des Untersuchungsbereichs 1 mit einem Anregungsfeld zu nutzen. In diesem Fall wird die Sekundärantenne 8 also nicht zum Empfang von Magnetresonanzsignalen genutzt.

Mittels der erfindungsgemäßen Magnetresonanzanlage ist auf einfache Weise trotz des hohen Grundmagnetfeldes im gesamten Untersuchungsobjekt 3 ein nahezu völlig homogenes Anregungsfeld realisierbar.

## Patentansprüche

1. Magnetresonanzanlage mit einem Grundfeldmagneten (4), einem Gradientensystem (6), einer Primärsendeantenne (7), mindestens einer Sekundärsendeantenne- (8), mindestens einer Empfangsantenne (7) und einer Steuer- und Auswerteeinrichtung (5),
- wobei ein Untersuchungsbereich (1) mittels des Grundfeldmagneten (4) mit einem statischen Grundmagnetfeld beaufschlagt wird,
- wobei der Untersuchungsbereich (1) mittels des Gradientensystems (6) mit Gradientenmagnetfeldern beaufschlagt wird,
- wobei der Untersuchungsbereich (1) mittels der mindestens einen Sendeantenne (7,8) mit hochfrequenten Anregungsfeldern beaufschlagt wird,
- wobei mittels der Empfangsantenne (7) Magnetresonanzsignale empfangen werden zu denen ein in den Untersuchungsbereich (1) einbringbares Untersuchungsobjekt (3) angeregt worden ist,
wobei das Gradientensystem (6) und die Sendeantennen (7,8) von der Steuer- und Auswerteeinrichtung (5) gesteuert werden, so dass im Gradientensystem (6) Gradientenströme und in den Sendeantennen (7,8) Anregungsströme fließen,
- wobei die Magnetresonanzsignale der Steuer- und Auswerteeinrichtung (5) zugeführt und von dieser ausgewertet werden,
wobei der Sekundärsendeantenne (8) mindestens em Steuerelement (10,11) zugeordnet ist, mittels dessen ein Amplitudenverhältnis und/oder ein Phasenversatz der Anregungsströme in der Primär- und in der Sekundärantenne zueinander eingestellt wird,
**dadurch gekennzeichnet,**
**dass** dieses Steuerelement von der Steuer- undAuswerteeinrichtung (5) so gesteuert wird, dass der in Sekundärsendeantenne (8) fließende Anregungsstrom eine kleinere Amplitude als der in der Primärsendeantenne (7) fließende Anregungsstrom aufweist und dass das von der Sekundärsendeantenne (8) erzeugte Anregungsfeld eine kleinere räumliche Erstreckung als das von der Primärsendeantenne (7) erzeugte Anregungsfeld aufweist.

2. Magnetresonanzanlage nach Anspruch 1, **dadurch gekennzeichnet, dass** das mindestens eine Steuerelement (10, 11) derart angesteuert wird, dass die Überlagerung der Anregungsfelder bei in den Untersuchungsbereich (1) eingebrachtem Untersuchungsobjekt (3) innerhalb des Untersuchungsobjekts (3) möglichst homogen ist.

3. Magnetresonanzanlage nach Anspruch 1 oder 2,
**dadurch gekennzeichnet,**
**dass** die Sekundärantenne (8) induktiv mit der Primärsendeantenne (7) gekoppelt ist.

4. Magnetresonanzanlage nach Anspruch 1, 2 oder 3, **dadurch gekennzeichnet, dass** der Steuer- und Auswerteeinrichtung (5) ein Steuersignal (S) vorgegeben wird, und dass die Ansteuerung des Steuerelements (10, 11) vom Steuersignal (S) abhängig ist.

5. Magnetresonanzanlage nach Anspruch 4, **dadurch gekennzeichnet, dass** die Sekundärsendeantenne (8) in Abhängigkeit vom Steuersignal (S) auch derart betrieben wird, dass sie während des Beaufschlagens des Untersuchungsbereichs (1) mit einem Anregungsfeld durch die Primärsendeantenne (7) bei einer Magnetresonanzfrequenz nicht resonant ist und den Untersuchungsbereich (1) nicht mit einem Anregungsfeld beaufschlagt.

6. Magnetresonanzanlage nach einem der obigen Ansprüche,
**dadurch gekennzeichnet,**
**dass** das Steuerelement (10, 11) als einstellbare Impedanz ausgebildet ist.

7. Magnetresonanzanlage nach einem der obigen Ansprüche, **dadurch gekennzeichnet, dass** der in der Sekundärsendeantenne (8) fließende Anregungsstrom derart gesteuert wird, dass er zum in der Primärsendeantenne (7) fließenden Anregungsstrom einen elektrischen Phasenversatz zwischen -180° und +180° aufweist.

8. Magnetresonanzanlage nach einem der Ansprüche 1 bis 7, **dadurch gekennzeichnet, dass** die Sekundärsendeantenne (8) mit der Steuer- und Auswerteeinrichtung (5) derart verbunden ist, dass sie nicht zum Empfang der Magnetresonanzsignale benützt wird.

9. Magnetresonanzanlage nach einem der Ansprüche 1 bis 7, **dadurch gekennzeichnet, dass** die Sekundärsendeantenne (8) mit der Steuer- und Auswerteeinrichtung (5) derart verbunden ist, dass sie auch zum Empfang der Magnetresonanzsignale benützt wird.

10. Magnetresonanzanlage nach Anspruch 9, **dadurch gekennzeichnet, dass** die Sekundärsendeantenne (8) zum Empfang von Magnetresonanzsignalen von der Steuer- und Auswerteeinrichtung (5) derart abgestimmt wird, dass sie bei einer Magnetresonanzfrequenz resonant ist.

## Claims

1. Magnetic resonance system with a basic field magnet (4), a gradient system (6), a primary transmit antenna (7), at least one secondary antenna (8), at least one receive antenna (7) and a control and evaluation facility (5),
- with an examination region (1) being charged with a static basic magnetic field by means of the basic field magnet (4),
- with the examination region (1) being charged with gradient magnetic fields by means of the gradient system (6),
- with the examination region (1) being charged with radio frequency excitation fields by means of the at least one transmit antenna (7, 8),
- with the receive antenna (7) being used to receive magnetic resonance signals, to which an examination subject (3) that can be introduced into the examination region (1) is excited,
- with the gradient system (6) and the transmit antennas (7, 8) being controlled by the control and evaluation facility (5) in such a manner that gradient currents flow in the gradient system (6) and excitation currents flow in the transmit antennas (7, 8),
- with the magnetic resonance signals being supplied to the control and evaluation facility (5) and being evaluated by this, with the secondary transmit antenna (8) being assigned at least one control element (10, 11), by means of which an amplitude ratio and/or a phase offset of the excitation currents in the primary antenna and the secondary antenna is/are adjusted in relation to each other,
**characterised in that**
this control element is controlled by the control and evaluation facility (5) in such a manner that the excitation current flowing in the secondary transmit antenna (8) has a smaller amplitude than the excitation current flowing in the primary transmit antenna (7) and the excitation field generated by the secondary transmit antenna (8) has a smaller spatial extent than the excitation field generated by the primary transmit antenna (7).

2. Magnetic resonance system according to claim 1, **characterised in that** the at least one control element (10, 11) is activated in such a manner that the superimposition of the excitation fields within the examination subject (3) is optimally homogenous when the examination subject (3) has been introduced into the examination region (1).

3. Magnetic resonance system according to claim 1 or 2, **characterised in that** the secondary antenna (8) is inductively coupled to the primary transmit antenna (7).

4. Magnetic resonance system according to claim 1, 2 or 3, **characterised in that** a control signal (S) is predetermined for the control and evaluation unit (5) and the activation of the control element (10, 11) is a function of the control signal (S).

5. Magnetic resonance system according to claim 4, **characterised in that** the secondary transmit antenna (8) is also operated as a function of the control signal (S) in such a manner that while the examination region (1) is being charged with an excitation field by the primary transmit antenna (7) it is not resonant at a magnetic resonance frequency and does not charge the examination region (1) with an excitation field.

6. Magnetic resonance system according to one of the preceding claims,
**characterised in that** the control element (10, 11) is configured as an adjustable impedance.

7. Magnetic resonance system according to one of the preceding claims, **characterised in that** the excitation current flowing in the secondary transmit antenna (8) is controlled in such a manner that it has an electrical phase offset between
-180° and +180° in relation to the excitation current flowing in the primary transmit antenna (7).

8. Magnetic resonance system according to one of claims 1 to 7, **characterised in that** the secondary transmit antenna (8) is connected to the control and evaluation facility (5) in such a manner that it is not used to receive magnetic resonance signals.

9. Magnetic resonance system according to one of claims 1 to 7, **characterised in that** the secondary transmit antenna (8) is connected to the control and evaluation facility (5) in such a manner that it is also used to receive magnetic resonance signals.

10. Magnetic resonance system according to claim 9, **characterised in that** the secondary transmit antenna (8) is tuned by the control and evaluation facility (5) to receive magnetic resonance signals in such a manner that it is resonant at a magnetic resonance frequency.

## Revendications

1. Appareil à résonance magnétique comprenant un aimant de champ de base (4), un système de gradients (6), une antenne émettrice primaire (7), au moins une antenne émettrice secondaire (8), au moins une antenne réceptrice (7) et un dispositif de commande et d'analyse (5),
- une zone d'étude (1) étant alimentée au moyen de l' aimant de champ de base (4) avec un champ magnétique de base statique,
- la zone d'étude (1) étant alimentée au moyen du système de gradients (6) avec des champs magnétiques de gradients,
- la zone d'étude (1) étant alimentée au moyen de l'au moins une antenne émettrice (7, 8) avec des champs d'excitation à haute fréquence,
- des signaux à résonance magnétique étant reçus au moyen de l'antenne réceptrice (7), avec lesquels un objet d'étude (3) pouvant être amené dans la zone d'étude (1) a été excité,
- le système de gradients (6) et les antennes émettrices (7, 8) étant commandés par le dispositif de commande et d'analyse (5), de sorte que des flux de gradients circulent dans le système de gradients (6) et des flux d'excitation circulent dans les antennes émettrices (7, 8),
- les signaux à résonance magnétique étant amenés au dispositif de commande et d'analyse (5) et étant analysés par celui-ci,
au moins un élément de commande (10, 11) étant attribué à l'antenne émettrice secondaire (8), élément au moyen duquel un rapport d'amplitudes et/ou un décalage de phase des flux d'excitation dans l'antenne primaire et dans l'antenne secondaire sont ajustés l'un par rapport à l'autre,
**caractérisé en ce que**
cet élément de commande est commandé par le dispositif de commande et d'analyse (5) de telle sorte que le flux d'excitation circulant dans l'antenne émettrice secondaire (8) présente une amplitude inférieure au flux d'excitation circulant dans l'antenne émettrice primaire (7) et que le champ d'excitation généré par l'antenne émettrice secondaire (8) présente une extension d'espace inférieure au champ d'excitation généré par l'antenne émettrice primaire (7).

2. Appareil à résonance magnétique selon la revendication 1, **caractérisé en ce que** l'au moins un élément de commande (10, 11) est commandé de telle sorte que la superposition des champs d'excitation sur l'objet d'étude (3) introduit dans la zone d'étude (1) à l'intérieur de l'objet d'étude (3) est la plus homogène possible.

3. Appareil à résonance magnétique selon la revendication 1 ou 2, **caractérisé en ce que** l'antenne secondaire (8) est couplée de façon inductive à l'antenne émettrice primaire (7).

4. Appareil à résonance magnétique selon la revendication 1, 2 ou 3, **caractérisé en ce qu'**un signal de commande (S) est attribué au dispositif de commande et d'analyse (5) et **en ce que** l'activation de l'élément de commande (10, 11) est dépendante du signal de commande (S).

5. Appareil à résonance magnétique selon la revendication 4, **caractérisé en ce que** l'antenne émettrice secondaire (8) est exploitée en fonction du signal de commande (S) également de telle sorte qu'elle n'est pas résonante pendant l'alimentation de la zone d'étude (1) avec un champ d'excitation par l'antenne émettrice primaire (7) avec une fréquence de résonance magnétique et n'alimente pas la zone d'étude (1) avec un champ d'excitation.

6. Appareil à résonance magnétique selon l'une quelconque des revendications précédentes, **caractérisé en ce que** l'élément de commande (10, 11) est conçu comme impédance réglable.

7. Appareil à résonance magnétique selon l'une quelconque des revendications précédentes, **caractérisé en ce que** le flux d'excitation circulant dans l'antenne émettrice secondaire (8) est commandé de telle sorte qu'il présente un décalage de phase électrique entre -180° et +180° par rapport au flux d'excitation circulant dans l'antenne émettrice primaire (7).

8. Appareil à résonance magnétique selon l'une quelconque des revendications 1 à 7, **caractérisé en ce que** l'antenne émettrice secondaire (8) est reliée au dispositif de commande et d'analyse (5) de telle sorte qu'elle n'est pas utilisée pour la réception des signaux à résonance magnétique.

9. Appareil à résonance magnétique selon l'une quelconque des revendications 1 à 7, **caractérisé en ce que** l'antenne émettrice secondaire (8) est reliée au dispositif de commande et d'analyse (5) de telle sorte qu'elle est utilisée également pour la réception des signaux à résonance magnétique.

10. Appareil à résonance magnétique selon la revendication 9, **caractérisé en ce que** l'antenne émettrice secondaire (8) est adaptée pour la réception de signaux à résonance magnétique par le dispositif de commande et d'analyse (5), de telle sorte qu'elle est résonante avec une fréquence de résonance magnétique.
